(19) Europäisches Patentamt
European Patent Office
Office européen des brevets

(11) Veröffentlichungsnummer: **0 482 508 B1**

(12) # EUROPÄISCHE PATENTSCHRIFT

(45) Veröffentlichungstag der Patentschrift: **12.07.95**

(51) Int. Cl.[6]: **C09B 44/10**, C07D 401/04, C07D 401/14

(21) Anmeldenummer: **91117713.7**

(22) Anmeldetag: **17.10.91**

(54) **Biskationische Azofarbstoffe sowie deren Zwischenprodukte.**

(30) Priorität: **26.10.90 DE 4034060**

(43) Veröffentlichungstag der Anmeldung:
**29.04.92 Patentblatt 92/18**

(45) Bekanntmachung des Hinweises auf die Patenterteilung:
**12.07.95 Patentblatt 95/28**

(84) Benannte Vertragsstaaten:
**AT CH DE FR GB IT LI SE**

(56) Entgegenhaltungen:
**DE-A- 3 538 517**

(73) Patentinhaber: **BASF Aktiengesellschaft**
**Carl-Bosch-Strasse 38**
**D-67063 Ludwigshafen (DE)**

(72) Erfinder: **Ruske, Manfred**
**Merziger Strasse 10**
**W-6700 Ludwigshafen (DE)**
Erfinder: **Mayer, Udo, Dr.**
**Max-Slevogt-Strasse 27**
**W-6710 Frakenthal (DE)**

**EP 0 482 508 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue biskationische Azofarbstoffe der Formel I

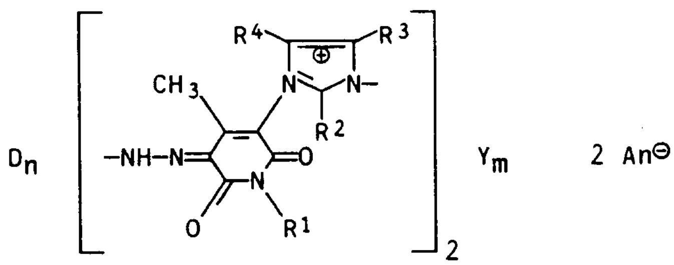

(I),

in der

| | |
|---|---|
| $R^1$ | Wasserstoff oder gegebenenfalls durch Phenyl substituiertes $C_1$-$C_5$-Alkyl, |
| $R^2$ | Wasserstoff oder $C_1$-$C_5$-Alkyl, |
| $R^3$ und $R^4$ | gleich oder verschieden sind und unabhängig voneinander jeweils Wasserstoff oder Methyl, |
| D | den Rest einer von Sulfonsäuregruppen freien aromatischen Diazokomponente, gegebenenfalls durch $C_1$-$C_4$-Alkyl oder $C_1$-$C_4$-Alkoxy substituiertes 1,4-Phenylen oder einen Rest der Formel 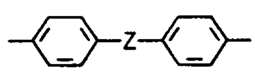 , worin Z für ein Brückenglied steht, |
| Y | Wasserstoff, $C_1$-$C_{18}$-Alkyl, $C_2$-$C_6$-Alkylen, Xyliden oder Phenylen, |
| $An^{\ominus}$ | das Äquivalent eines Anions, |
| m | 1 oder 2 und |
| n | 1 oder 2 bedeuten, |

mit der Maßgabe, daß

a) wenn Y Wasserstoff oder $C_1$-$C_{18}$-Alkyl bedeutet, m für 2, n für 1 und D für gegebenenfalls durch $C_1$-$C_4$-Alkyl oder $C_1$-$C_4$-Alkoxy substituiertes 1,4-Phenylen oder einen Rest der Formel

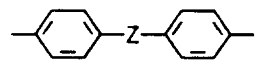

stehen, und

b) wenn Y $C_2$-$C_6$-Alkylen, Xyliden oder Phenylen bedeutet, m für 1, n für 2 und D für den Rest einer von Sulfonsäuregruppen freien aromatischen Diazokomponente stehen,

deren Zwischenprodukte sowie die Verwendung der neuen Farbstoffe zum Färben oder Bedrucken von polymerem Material.

Aus der DE-A-2 054 697, DE-A-2 627 680, DE-A-3 538 517 und EP-A 92 520 sind bereits kationische Azofarbstoffe bekannt.

Aufgabe der vorliegenden Erfindung war es, neue biskationische Azofarbstoffe bereitzustellen, die über vorteilhafte anwendungstechnische Eigenschaften verfügen.

Demgemäß wurden die eingangs näher bezeichneten biskationischen Azofarbstoffe der Formel I gefunden.

Die biskationischen Azofarbstoffe der Formel I können in verschiedenen tautomeren Formen vorliegen, die alle vom Patentanspruch umfaßt werden.

Alle in der obengenannten Formel I auftretenden Alkyl- und Alkylengruppen können sowohl geradkettig als auch verzweigt sein.

Reste $R^1$, $R^2$ und Y sind z.B. Methyl, Ethyl, Propyl, Isopropyl, Butyl, Isobutyl, sec-Butyl, tert-Butyl, Pentyl, Isopentyl, Neopentyl oder tert-Pentyl.

Reste $R^1$ sind weiterhin z.B. Benzyl oder 1- oder 2-Phenylethyl.

Reste Y sind weiterhin z.B. Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Methylen, Ethylen, 1,2- oder 1,3-Propylen, 1,2-, 1,3-, 1,4- oder 2,3-Butylen, 1,5-Pentylen oder 1,6-Hexylen.

Wenn D den Rest einer von Sulfonsäuregruppen freien aromatischen Diazokomponente bedeutet, leiten sich die freien Amine der Formel $D\text{-}NH_2$ beispielsweiseaus der Anilin-, Naphthalin-, Aminoanthrachinon-, Aminothiazol-, Aminobenzthiazol- oder Aminotriazolreihe ab.

Die genannten Amine $D\text{-}NH_2$ können dabei ein- oder mehrfach, in der Regel ein- bis dreifach, substituiert sein, wobei als geeignete Substituenten z.B.Halogen, wie Chlor oder Brom, Nitro, $C_1$-$C_4$-Alkoxy, Benzyloxy, Phenoxy, Halogenphenoxy, $C_1$-$C_4$-Alkyl, Phenyl, Trifluormethyl, Carbamoylmethyl, N-Phenylcarbamoylmethyl, $C_1$-$C_4$-Alkanoyl, Benzoyl, Mono- oder Di-$C_1$-$C_4$-alkylbenzoyl, Mono- oder Di-$C_1$-$C_4$-alkoxybenzoyl, ($C_1$-$C_4$-Alkyl)-($C_1$-$C_4$-alkoxy)benzoyl, Dimethylaminoacetyl, $C_1$-$C_4$-Alkoxycarbonyl, Carbamoyl, $C_1$-$C_4$-Mono- oder Dialkylcarbamoyl, Cyano, $C_1$-$C_4$-Alkanoylamino, Benzoylamino, Phthalimido, Phenylamino, Mono- oder Dinitrophenylamino, $C_1$-$C_4$-Alkylsulfonyl, Phenoxysulfonyl, Sulfamoyl, $C_1$-$C_4$-Mono- oder Dialkylsulfamoyl, Phenylsulfamoyl, N-($C_1$-$C_4$-Alkyl)-N-phenylsulfamoyl, Halogenphenylsulfamoyl, Naphthylsulfamoyl, Phenylazo, Nitrophenylazo, $C_1$-$C_4$-Alkanoylaminophenylazo oder $C_1$-$C_4$-Dialkylaminophenylazo in Betracht kommen können.

Beispielhaft seien folgende Amine genannt:

Anilin, 1-Amino-2-chlorbenzol, 1-Amino-3-chlorbenzol, 1-Amino-2,6-dichlorbenzol, 1-Amino-2,3-dichlorbenzol, 1-Amino-2,5-dichlorbenzol, 1-Amino-2,4-dichlorbenzol, 1-Amino-3,4-dichlorbenzol, 1-Amino-3,5-dichlorbenzol, 1-Amino-4-acetaminobenzol, 1-Amino-2-chlor-4-acetaminobenzol, 1-Amino-4-benzoylaminobenzol, 1-Amino-4-phenylbenzol, 4-Amino-1,1'-diphenylether, 4-Amino-4'-chlor-1,1'-diphenylether, 2-Amino-4'-chlor-1,1'-diphenylether, 2-Amino-1,1'-diphenylether, 1-Amino-2-chlor-4-methylsulfonylbenzol, 1-Amino-4-methylsulfonylbenzol, 1-Aminobenzol-3-sulfonsäurephenylester, 1-Aminobenzol-4-sulfonsäurephenylester, 1-Amino-2-chlorbenzol-5-sulfonsäurephenylester, 1-Amino-2-methylbenzol-5-sulfonsäurephenylester, 1-Amino-2,6-dichlorbenzol-4-sulfonsäurephenylester, 1-Amino-3-trifluormethylbenzol, 1-Amino-3,5-bis(trifluormethyl)-benzol, 1-Amino-2-trifluormethyl-4-chlorbenzol, 1-Amino-4-ethoxycarbonylaminobenzol, 1-Amino-2,5-dimethoxy-4-ethoxycarbonylaminobenzol, 1-Aminonaphthalin, 1-Aminonaphthalin-4-sulfonsäuredimethylamid, 4-Aminophenylphthalimid, 2-Aminoterephthalsäuredimethylester, 4-Aminobenzophenon, 4-Amino-4'-methylbenzophenon, 1-Amino-4-acetylbenzol, 4-Amino-2',4'-dinitrodiphenylamin, 4-Amino-4'-nitrodiphenylamin, 4-Amino-2'-nitrodiphenylamin, 1-Amino-2-methylbenzol, 1-Amino-3-methylbenzol, 1-Amino-4-methylbenzol, 1-Amino-2,5-dimethylbenzol, 1-Amino-2-methyl-3-chlorbenzol, 1-Amino-2-methyl-6-chlorbenzol, 1-Amino-2-methyl-5-chlorbenzol, 1-Amino-2-chlor-4-methylbenzol, 1-Amino-3-chlor-4-methylbenzol, 1-Amino-3,6-dichlor-4-methylsulfamoylbenzol, 1-Amino-2-chlor-4-nitrobenzol, 1-Amino-2-brom-4-nitrobenzol, 1-Amino-2,5-dichlor-4-methoxybenzol, 1-Amino-2-methoxybenzol, 1-Amino-4-methoxybenzol, 1-Amino-2,5-dimethylbenzol, 1-Amino-2-methyl-3-chlorbenzol, 1-Amino-2-methyl-6-chlorbenzol, 1-Amino-2-methyl-5-chlorbenzol, 1-Amino-2-chlor-4-methylbenzol, 1-Amino-3-chlor-4-methylbenzol, 1-Amino-3,6-dichlor-4-dimethylsulfamoylbenzol, 1-Amino-2-chlor-4-nitrobenzol, 1-Amino-2-brom-4-nitrobenzol, 1-Amino-2,5-dichlor-4-methoxybenzol, 1-Amino-2-methoxybenzol, 1-Amino-4-methoxybenzol, 1-Amino-3-chlor-4-methoxybenzol, 1-Amino-2-ethoxybenzol, 1-Amino-4-ethoxybenzol, 1-Amino-4-benzyloxybenzol, 1-Aminophenylacetamid, 1-Aminophenylacetanilid, 1-Amino-2-cyanobenzol, 1-Amino-4-cyanobenzol, 1-Amino-3-cyanobenzol, 1-Amino-2-cyano-4-nitrobenzol, 1-Amino-2-cyano-4-nitro-6-brombenzol, 1-Amino-2-nitro-4-methylbenzol, 1-Amino-2-nitro-4-chlorbenzol, 1-Amino-2,5-dimethoxy-4-chlorbenzol, 1-Amino-2,5-dimethoxy-4-brombenzol, 1-Amino-2,5-dimethoxybenzol, 1-Amino-2,5-diethoxybenzol, 1-Amino-2,5-dimethoxy-4-acetaminobenzol, 1-Amino-2,5-dimethoxy-4-benzoylaminobenzol, 1-Amino-2,5-diethoxy-4-benzoylaminobenzol, 1-Aminobenzol-4-sulfonsäuremethylamid, 1-Aminobenzol-4-sulfonsäuredimethylamid, 1-Aminobenzol-4-sulfonsäurephenylamid, 1-Aminobenzol-4-sulfonsäure-2'-chlorphenylamid, 1-Aminobenzol-3-sulfonsäurephenylamid, 1-Aminobenzol-2-sulfonsäure-N-ethyl-N-phenylamid, 1-Amino-2-methyl-5-sulfamoylbenzol, 1-Amino-4-methylbenzol-3-sulfonsäure-N-ethyl-N-phenylamid, 1-Amino-4-methylbenzol-3-sulfonsäurephenylamid, 1-Amino-2,5-dichlorbenzol-4-sulfonsäurephenylamid, 1-Amino-3-nitrobenzol, 1-Amino-4-nitrobenzol, 1-Amino-2-methoxy-4-nitrobenzol, 1-Amino-2,5-dimethoxy-4-nitrobenzol, 1-Amino-2,5-dinitrobenzol, 1-Aminobenzol-4-carbonsäuremethylester, 1-Aminobenzol-4-carbonsäureethylester, 1-Amino-2-methylbenzol-4-carbonsäureethylester, 1-Amino-2-methylbenzol-4-carbonsäurephenylamid, 1-Aminobenzol-4-carbonsäurephenylamid, 1-Aminobenzol-4-carbonsäuredimethylamid, 2-Aminobenzamid, 1-Amino-4-chlorbenzol-2-carbonsäuremethylester, 1-Aminobenzol-2-carbonsäuremethylester, 1-Aminobenzol-3-carbonsäuremethylester, 1-Aminobenzol-3-carbonsäurephenylamid, 1-Amino-3-nitro-4-methylbenzol, 1-Amino-2-nitro-4-methoxybenzol, 1-Amino-3-acetaminobenzol, 1-Aminobenzol-4-sulfonsäurenaphth-1-ylamid, 1-Amino-2-nitro-4-methylbenzol, 1-Amino-2,4,6-trichlorbenzol, 1-Amino-4-(dimethylaminoacetyl)benzol, 1-Amino-2-nitro-4-acetaminobenzol, 4-Aminoazobenzol, 4-Amino-2-methyl-5-methoxyazobenzol, 4-Amino-2,5-dimethoxyazobenzol, 4-Amino-4'-nitroazobenzol, 4-Amino-4'-acetaminoazobenzol, 4-Amino-4'-dimethylaminoazobenzol, 1-Amino-4'-phenylazonaphthalin, 1-Amino-2-ethoxy-4' -phenylazonaphthalin, 4-Amino-3'-methylazobenzol, 4-Amino-4'-methoxyazobenzol, 2-Aminoanthrachinon, 2-Aminothiazol, 2-Amino-5-methoxybenzthiazol, 2-Aminobenzthiazol-6-sulfonamid oder 3-Amino-1,2,4-triazol.

Wenn D einen Rest der Formel

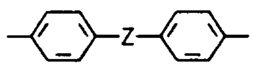

bedeutet, so sind geeignete Brückenglieder Z beispielsweise $C_2$-$C_6$-Alkylen, $C_2$-$C_6$-Alkylenoxy oder ein Rest der Formel -CH = CH-, -NH-CO-, -N = N-, -NH-CO-NH-,

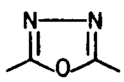 , 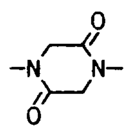 oder 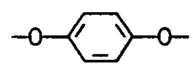 .

Geeignete Anionen $An^{\ominus}$ sind z.B. Fluorid, Chlorid, Bromid, Iodid, Formiat, Acetat, Propionat, Mono-, Di- oder Trichloracetat, Hydroxyacetat, Methoxyacetat, 2-Hydroxyethoxyacetat, Lactat, Citrat, Succinat, Glutarat, Methylsulfonat, Phenylsulfonat oder 2- oder 4-Methylphenylsulfonat.

Wenn D den Rest einer von Sulfonsäuregruppen freien aromatischen Diazokomponente bedeutet, so sind solche biskationischen Azofarbstoffe der Formel I bevorzugt, in der D für den Rest einer Diazokomponente aus der Anilinreihe steht.

Hervorzuheben sind biskationische Azofarbstoffe der Formel I, in der

Y Wasserstoff oder $C_1$-$C_5$-Alkyl,
D Phenylen oder einen Rest der Formel

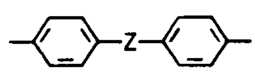

worin Z die obengenannte Bedeutung besitzt,
m 2 und
n 1 bedeuten.

Weiterhin hervorzuheben sind biskationische Azofarbstoffe der Formel I, in der

Y $C_2$-$C_6$-Alkylen, Xyliden oder Phenylen,
D den Rest einer von Sulfonsäuregruppen freien Diazokomponente aus der Anilinreihe,
m 1 und
n 2 bedeuten.

Die vorliegende Erfindung betrifft weiterhin verdoppelte Imidazolylpyridone der Formel II

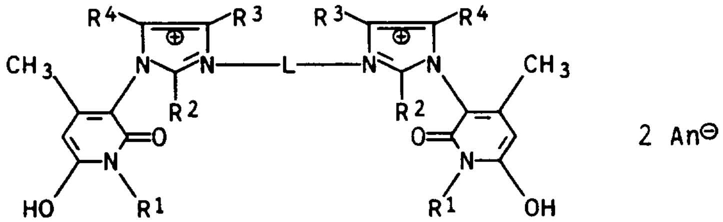

(II),

in der

L $C_2$-$C_6$-Alkylen, Xyliden oder Phenylen,

$R^1$ Wasserstoff oder gegebenenfalls durch Phenyl substituiertes $C_1$-$C_5$-Alkyl,

$R^2$ Wasserstoff oder $C_1$-$C_5$-Alkyl,

$R^3$ und $R^4$ gleich oder verschieden sind und unabhängig voneinander jeweils Wasserstoff oder Methyl und

$An^{\ominus}$ das Äquivalent eines Anions bedeuten.

Bezüglich der beispielhaften Aufzählung der in Formel II auftretenden Reste sei auf die oben vorgenommene Aufzählung verwiesen.

Die Herstellung der neuen biskationischen Farbstoffe der Formel I kann in an sich bekannter Weise geschehen. Beispielsweise kann man ein Amin der Formel IIIa

$D^1$-$NH_2$ (IIIa),

in der $D^1$ den Rest einer von Sulfonsäuregruppen freien aromatischen Diazokomponente bedeutet, diazotieren und mit einem verdoppelten Imidazolylpyridon der Formel II kuppeln.

Man kann auch ein Diamin der Formel IIIb

$H_2N$-$D^2$-$NH_2$ (IIIb),

in der $D^2$ gegebenenfalls durch $C_1$-$C_4$-Alkyl oder $C_1$-$C_4$-Alkoxy substituiertes 1,4-Phenylen oder einen Rest der Formel

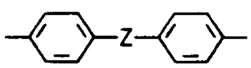

bedeutet, worin Z die obengenannte Bedeutung besitzt, tetrazotieren und mit einem Imidazolylpyridon der Formel IV

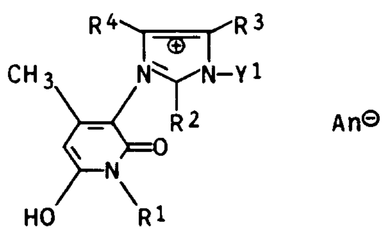

(IV),

in der $R^1$, $R^2$, $R^3$, $R^4$ und $An^{\ominus}$ jeweils die obengenannte Bedeutung besitzen und $Y^1$ Wasserstoff oder $C_1$-$C_{18}$-Alkyl bedeutet, kuppeln.

Die Diazotierung der Amine der Formel IIIa und die Tetrazotierung der Diamine der Formel IIIb können, ebenso wie die Kupplung mit den Kupplungskomponenten der Formel II und IV, nach bekannten Methoden durchgeführt werden.

Vorteilhaft kuppelt man dabei jeweils in wäßrigem, saurem oder neutralem bis schwach alkalischem Medium in Gegenwart von Puffern, wie den Alkalisalzen der Ameisen-, Essig- oder Propionsäure, den Alkalisalzen von Dicarbonsäuren, wie Bernstein-, Glutar- oder Adipinsäure, den Alkalisalzen der Phosphorsäure oder Kohlensäure oder deren Gemische.

Die Isolierung des Farbstoffs erfolgt zweckmäßig als Farbbase bei einem pH-Wert oberhalb von 9. Es ist jedoch auch möglich, die Reaktionslösung direkt weiterzuverarbeiten.

Zu ihrer Anwendung werden die Farbbasen zweckmäßig durch Lösen in verdünnten, wäßrigen Säuren, wie Ameisensäure, Essigsäure, Propionsäure, Milchsäure, Citronensäure, Glykolsäure, Diglykolsäure, Bernsteinsäure, Glutarsäure, Methansulfonsäure, Amidosulfonsäure oder in Mischungen dieser Säuren, in die leicht wasserlöslichen 4-Methyl-6-hydroxypyrid-2-on-3-imidazolium-salze übergeführt und so in eine lagerstabile, flüssige Form gebracht.

Zur Erhöhung des Fließverhaltens und der Lagerstabilität können Zusätze von Glykolen, wie Diethylenglykol oder Triethylenglykol, Glykolethern, Harnstoff, Formamid oder N-Methylpyrrolidinon erfolgen.

Die Imidazolylpyridone der Formel IV werden ebenfalls nach bekannten Verfahren, beispielsweise durch Kondensation eines Imidazoliumchlorid-acetamids mit Acetessigsäuremethylester in alkoholischer Natriummethylatlösung erhalten.

Aus den Imidazolylpyridonen der Formel IV können beispielsweise durch Umsetzung mit einer Verbindung der Formel V

X-L-X (V),

in der L die obengenannte Bedeutung besitzt und X für eine Austrittsgruppe (z.B. Chlor, Brom, Iod, Methosulfat, Ethosulfat, Benzolsulfonat oder Toluolsulfonat) steht, die verdoppelten Imidazolylpyridone der Formel II hergestellt werden.

Eine bevorzugte Herstellungsweise für die Imidazolylpyridone der Formel II ist jedoch die Kondensation von Diimidazolen der Formel VI

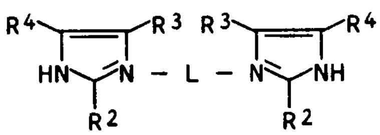 (VI),

in der L, $R^2$, $R^3$ und $R^4$ jeweils die obengenannte Bedeutung besitzen, mit Chloracetamid oder N-($C_1$-$C_5$-Alkyl)chloracetamiden zu Verbindungen der Formel VII

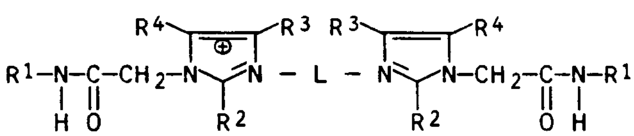 (VII),

in der L, $R^1$, $R^2$, $R^3$, $R^4$ und $An^\ominus$ jeweils die obengenannte Bedeutung besitzen. Diese können dann in einer Knoevenagel-Reaktion in Gegenwart von basischen Katalysatoren zu den Imidazolylpyridonen der Formel II umgesetzt werden.

Die Herstellung der Diimidazole VI ist beisielsweise in der DE-A-2 903 653, in J. prakt. Chem., Band 280, Seiten 306 bis 313, 1959, oder in Chem. Heterocycl. Comp., Band 6, Seiten 194 bis 197, 1970, beschrieben.

Die erfindungsgemäßen biskationischen Azofarbstoffe der Formel I können für sich alleine, in Gemischen untereinander oder zusammen mit anderen kationischen oder anionischen Verbindungen in Form ihrer Lösungen oder in Form von Pulvern oder Granulaten angewendet werden.

Sie eignen sich vorteilhaft zum Färben oder Bedrucken von polymerem Material, insbesondere von Papierstoffen, aber auch von Cellulose, Baumwolle, Leder, Bastfasern, Hanf, Flachs, Sisal, Jute, Kokos oder Stroh.

Die Farbstoffe können vorzugsweise bei der Herstellung von in der Masse gefärbtem, geleimtem und ungeleimtem Papier eingesetzt werden. Sie können ebenfalls zum Färben von Papier nach dem Tauchverfahren angewendet werden.

Das Färben von Papier, Leder oder Cellulose erfolgt nach an sich bekannten Methoden.

Die neuen Farbstoffe oder ihre Präparationen färben das Abwasser bei der Papierherstellung praktisch gar nicht oder nur wenig an, was für die Reinhaltung der Gewässer besonders günstig ist. Sie sind hoch substantiv, melieren auf Papier gefärbt nicht und sind weitgehend pH-unempfindlich. Die Färbungen auf Papier zeichnen sich durch eine gute Lichtechtheit aus. Nach längerem Belichten ändert sich die Nuance Ton-in-Ton.

Die gefärbten Papiere sind naßecht, nicht nur gegen Wasser, sondern ebenfalls gegen Milch, Seifenwasser, Natriumchloridlösungen, Fruchtsäfte oder gesüßte Mineralwasser und wegen ihrer guten Alkoholechtheit auch gegen alkoholische Getränke beständig.

Mit den neuen Farbstoffen kann man auch Polyacrylnitriltextilien oder durch anionische Gruppen modifizierte Polyamid- oder Polyestertextilien färben, foulardieren oder bedrucken.

Die folgenden Beispiele sollen die Erfindung näher erläutern. Die dort angegebenen Prozente sind jeweils Gewichtsprozente.

Beispiel 1

5,3 g 4,4'-Diaminodibenzyl, 35 ml Wasser und 20,4 g 30 %ige Salzsäure wurden mit 100 g Eis versetzt und bei 0 bis 5 °C mit 3,5 g Natriumnitrit, gelöst in Wasser, in bekannter Weise tetrazotiert. Nach Beseitigung des Nitritüberschusses mit Amidosulfonsäure wurde die kalte Lösung des Tetrazoniumchlorids gleichzeitig mit einer Lösung von 13,5 g 3-(3-Propylimidazolium)-4-methyl-6-hydroxypyrid-2-on-chlorid in 175 ml Wasser und 2 g Essigsäure, in 200 g 10 %ige Natriumacetatlösung und etwas Eis eingetragen. Das Reaktionsgemisch wurde eine Stunde bei einem pH-Wert von 4 bis 5 und bei 20 bis 22 °C bis zur vollständigen Kupplung gerührt. Die Reaktionsmischung wurde dann mit 10 %iger Sodalösung auf einen pH-Wert von 9,5 gestellt und kurzzeitig auf 80 °C erhitzt.

Der gut ausgeflockte Niederschlag der Farbbase der Formel

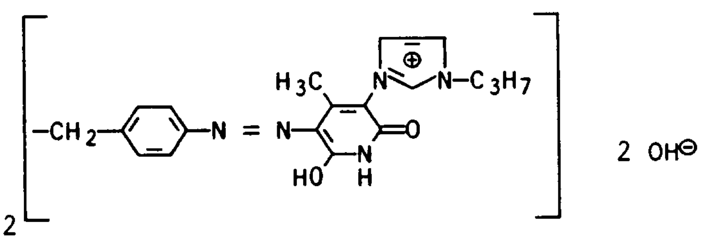

wurde abfiltriert, mit Wasser gewaschen und getrocknet. Es wurden 17,7 g der Farbbase erhalten. Die Lösung der Farbbase als Acetat in wäßriger Essigsäure hat ein Absorptionsmaximum ($\lambda_{max}$) von 442,1 nm. In der Tissuemassefärbung ist der Farbstoff auch bei Neutralbedingungen sehr affin. Die Ausblutechtheiten der gelben Papierfärbungen jeweils in Kondenswasser, 1,5 %iger Essigsäure, 0,5 %iger Sodalösung und 50 %igem Ethanol sind sehr gut.

Analog Beispiel 1 können die in Tabelle 1 aufgeführten Farbstoffe erhalten werden.

Tabelle 1

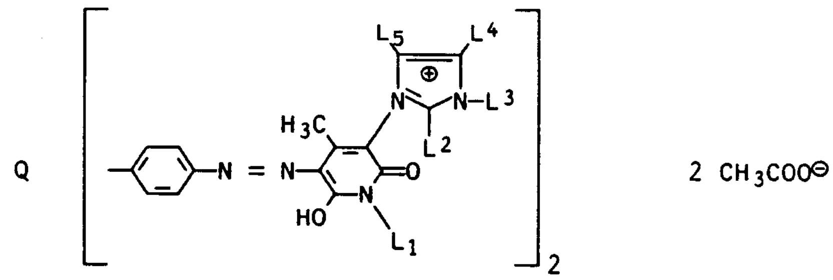

| Bsp. Nr. | Q | $L_1$ | $L^2$ | $L^3$ | $L^4$ | $L_5$ | $\lambda_{max}$ [nm] |
|---|---|---|---|---|---|---|---|
| 2 | $-CH_2-CH_2-$ | $CH_3$ | H | $CH_3$ | H | H | 441,1 |
| 3 | $-CH_2-CH_2-$ | H | H | $CH_3$ | H | H | 441,1 |
| 4 | $-CH_2-CH_2-$ | H | H | i-Propyl | H | H | 441,1 |
| 5 | $-CH_2-CH_2-$ | H | $CH_3$ | $CH_3$ | H | H | 442,1 |
| 6 | $-CH_2-CH_2-$ | H | H | $CH_3$ | H | $CH_3$ | 442,1 |
| 7 | $-CH_2-CH_2-$ | H | H | $CH_3$ | $CH_3$ | H | 441,1 |
| 8 | -NH-CO- | $CH_3$ | H | $CH_3$ | H | H | 456,1 |
| 9 | -NH-CO- | $CH_3$ | H | $C_2H_5$ | H | H | 448,1 |
| 10 | -NH-CO- | $CH_3$ | H | $C_3H_7$ | H | H | 457,1 |
| 11 | -NH-CO- | $CH_3$ | H | $CH_3$ | H | $CH_3$ | 453,1 |
| 12 | -NH-CO- | $CH_3$ | H | i-Propyl | H | H | 457,1 |
| 13 | -NH-CO- | H | H | $CH_3$ | H | H | 454,1 |
| 14 | -NH-CO- | H | H | $C_2H_5$ | H | H | 448,1 |
| 15 | -NH-CO- | H | H | $C_3H_7$ | H | H | 457,1 |
| 16 | -NH-CO- | H | H | i-Propyl | H | H | 453,1 |
| 17 | -NH-CO- | H | $CH_3$ | $CH_3$ | H | H | 458,1 |
| 18 | -NH-CO- | H | H | $CH_3$ | H | $CH_3$ | 456,1 |
| 19 | -N=N- | H | $CH_3$ | $CH_3$ | H | H | 500,1 |
| 20 | $-O(CH_2)_3-$ | H | $CH_3$ | $CH_3$ | H | H | 447,1 |
| 21 | -NH-CO-NH- | H | H | $CH_3$ | H | H | 478,1 |
| 22 | -NH-CO-NH- | H | H | $C_2H_5$ | H | H | 476 |
| 23 | -NH-CO-NH- | H | H | $C_3H_7$ | H | H | 481,1 |
| 24 | -NH-CO-NH- | H | H | i-Propyl | H | H | 478,1 |
| 25 | -NH-CO-NH- | H | $CH_3$ | $CH_3$ | H | H | 480,1 |
| 26 | -NH-CO-NH- | H | H | $CH_3$ | H | $CH_3$ | 479 |

Fortsetzung Tabelle 1

| Bsp. Nr. | Q | $L_1$ | $L^2$ | $L^3$ | $L^4$ | $L_5$ | $\lambda_{max}$ [nm] |
|---|---|---|---|---|---|---|---|
| 27 | -NH-CO-NH- | $CH_3$ | H | $CH_3$ | H | H | 476,1 |
| 28 | -NH-CO-NH- | $CH_3$ | H | $C_2H_5$ | H | H | 476,1 |
| 29 | -NH-CO-NH- | $CH_3$ | H | $C_3H_7$ | H | H | 481,1 |
| 30 | -NH-CO-NH- | $CH_3$ | H | i-Propyl | H | H | 480,1 |
| 31 | -NH-CO-NH- | $CH_3$ | H | H | H | $CH_3$ | 478,1 |
| 32 | -N=N- | H | H | $CH_3$ | H | H | 495 |

Beispiel 33

14,25 g 3-(3-Methylimidazolium)-4-methyl-5-[azo-(4-aminophenyl)]-1-methylpyrid-2,6-dion-chlorid (hergestellt durch salzsaure Hydrolyse des 4'-Acetylaminobenzols) wurden in 50 ml Wasser und 20 g 30 %iger Essigsäure gelöst und nach Zugabe von Eis bei 0 bis 5 °C mit 2,8 g Natriumnitrit in bekannter Weise diazotiert. Das Diazoniumchlorid wurde mit einer Lösung von 10,26 g 2-(3-Methylimidazolium)-1,4-dimethylpyrid-2,6-dion-chlorid in 120 ml Wasser und 2 g 30 %iger Essigsäure in Gegenwart von 30 g 10 %iger Natriumacetatlösung bei einem pH-Wert von 4 bis 5 und bei 5 bis 15 °C umgesetzt.

Die Farbbase wurde bei einem pH-Wert von 9,5 mit Sodalösung ausgefällt, auf 60 °C erwärmt, filtriert, mit Wasser salzfrei gewaschen und getrocknet. Es wurden 10,3 g eines roten Farbstoffs der Formel

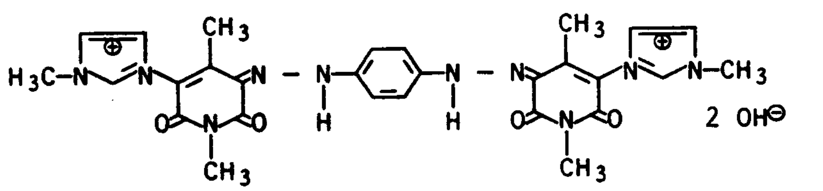

erhalten. $\lambda_{max}$ (Essigsäure): 513,1 nm.

Bei einem pH von 4 bis 5 wird die Papiermasse sehr farbstark in blaustichig rotem Farbton gefärbt.

Analog Beispiel 33 können die in der folgenden Tabelle 2 aufgeführten Farbstoffe der Formel

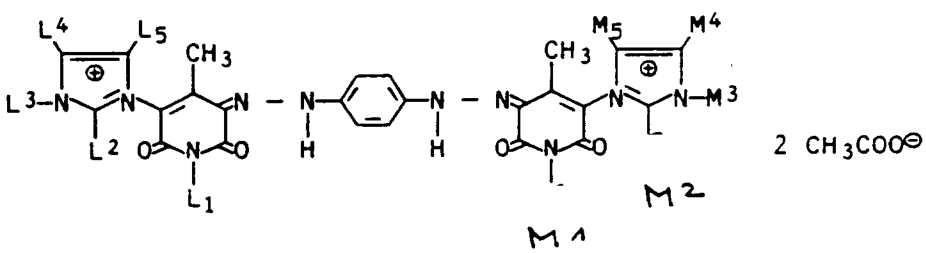

erhalten werden.

Tabelle 2

| Bsp. Nr. | $L_1$ | $L^2$ | $L^3$ | $L^4$ | $L^5$ | $M^1$ | $M^2$ | $M^3$ | $M^4$ | $M_5$ | $\lambda_{max}$ [nm] |
|---|---|---|---|---|---|---|---|---|---|---|---|
| 34 | H | H | $C_3H_7$ | H | H | H | H | $C_3H_7$ | H | H | 514,1 |
| 35 | H | H | $C_3H_7$ | H | H | $CH_3$ | H | $CH_3$ | H | H | 514,1 |
| 36 | $CH_3$ | H | $CH_3$ | H | H | $CH_3$ | H | $CH_3$ | H | H | 513,1 |
| 37 | H | H | $CH_3$ | H | H | $CH_3$ | H | $CH_3$ | H | H | 514,1 |
| 38 | H | H | $CH_3$ | H | H | H | H | $CH_3$ | H | H | 514,1 |
| 39 | H | H | $CH_3$ | H | H | H | H | $C_3H_7$ | H | H | 515,1 |

Beispiel 40

7,5 g 1-Amino-4-acetylaminobenzol wurden in 45 g Eiswasser fein suspendiert und bei 0 bis 5 °C mit 25 g 18 %iger Salzsäure versetzt. Nach weiterer Eiszugabe wurden 3,45 g Natriumnitrit in wäßriger Lösung Zugesetzt. Nach etwa 30 Minuten wurde das gebildete Diazoniumchlorid zu einer sehr feinteiligen Suspension von 12 g 1,2-Bis(4-methyl-6-hydroxypyrid-2-on-3-imidazolium)ethan-chlorid in 150 ml Wasser und 6,5 g Essigsäure eingetragen. Nach beendeter Kupplung wurde das Gemisch auf einen pH-Wert von 9 gestellt und auf 80 °C erwärmt. Bei 25 °C wurde die Fällungssuspension filtriert und mit Wasser salzfrei gewaschen. Es wurden 16,8 g einer Farbbase der Formel

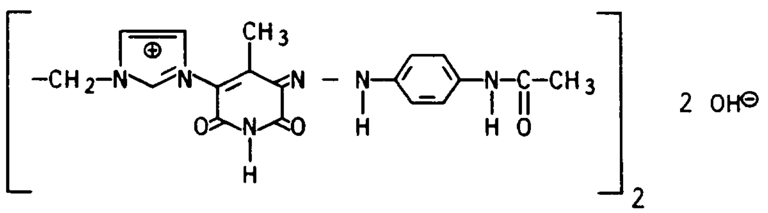

erhalten. $\lambda_{max}$ (Essigsäure): 448,1 nm.

Beispiel 41

Anstelle von 1-Amino-4-acetylaminobenzol wurden in Beispiel 40 3,2 g 1-Amino-4-benzoylaminobenzol verwendet. Man erhielt 21,3 g eines gelben Farbstoffs der Formel

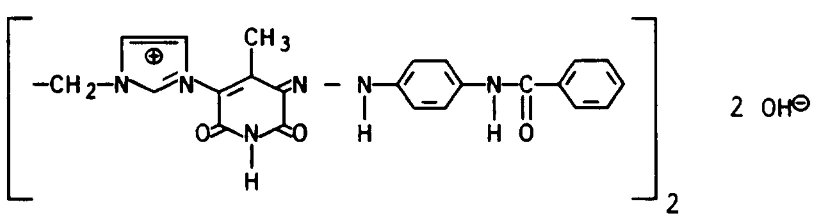

$\lambda_{max}$ (Essigsäure): 447,1 nm. Der Farbstoff hat eine sehr hohe Faseraffinität zu Papier.

Beispiel 42

Anstelle von 1-Amino-4-acetylaminobenzol wurden in Beispiel 40 6,1 g p-Anisidin verwendet. Man erhielt 11,2 g einer gelben Farbbase der Formel

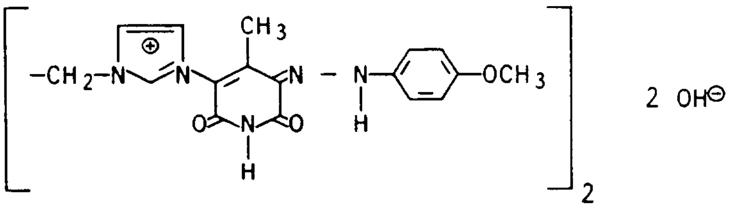

$\lambda_{max}$ (Essigsäure): 455,1.

Werden aromatische Amine der Formel Q-$NH_2$ nach bekannten Verfahren diazotiert und mit 1,2-Bis(4-methyl-6-hydroxypyrid-2-on-imidazolium)ethan-chlorid gekuppelt, werden Farbbasen der allgemeinen Formel

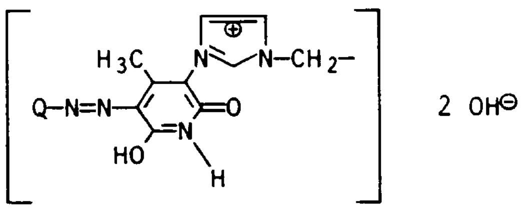

erhalten, die die in der Tabelle 3 angegebenen Extinktionwerte, gemessen in 10 %iger Essigsäure, aufweisen.

Tabelle 3

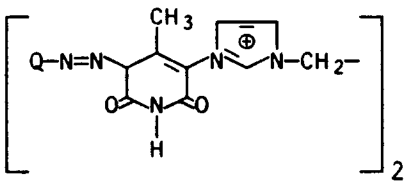

2 $CH_3COO^{\ominus}$

| Bsp. Nr. | Q | $\lambda_{max}$ [nm] |
|---|---|---|
| 43 | 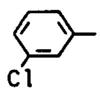 | 420 |
| 44 | 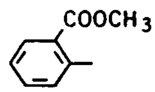 | 422 |
| 45 | 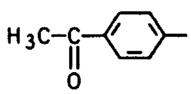 | 425 |
| 46 | 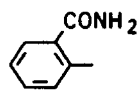 | 425 |
| 47 | 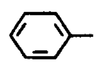 | 426 |
| 48 | 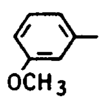 | 429 |
| 49 | 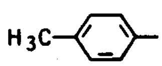 | 436,1 |
| 50 | 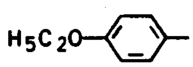 | 451 |
| 51 | 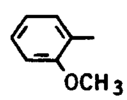 | 451 |
| 52 | 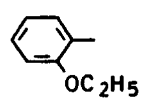 | 451 |
| 53 | 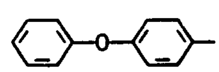 | 442 |
| 54 | 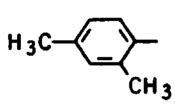 | 444 |

Tabelle 3 (Fortsetzung)

| Bsp. Nr. | Q | $\lambda_{max}$ [nm] |
|---|---|---|
| 55 | 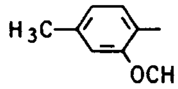 | 460 |
| 56 | 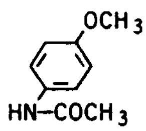 | 462 |
| 57 | 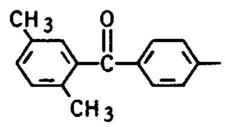 | 426,1 |
| 58 | 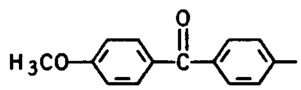 | 426,1 |
| 59 | 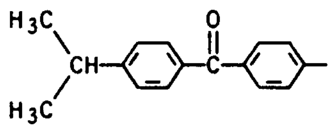 | 425,1 |
| 60 | 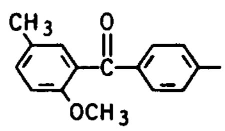 | 427,1 |
| 61 | 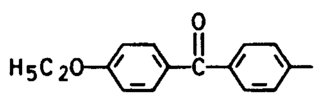 | 426,1 |
| 62 | 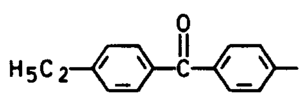 | 425,1 |
| 63 | 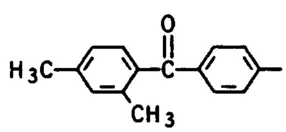 | 427,1 |
| 64 | 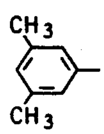 | 433,1 |
| 65 | 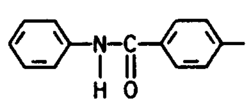 | 429,1 |
| 66 | 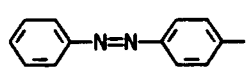 | 449,1 |

Tabelle 3 (Fortsetzung)

| Bsp. Nr. | Q | $\lambda_{max.}$ [nm] |
|---|---|---|
| 67 | 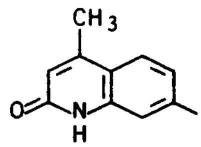 | 442,1 |
| 68 | 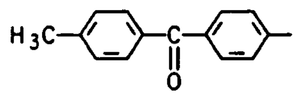 | 424,1 |

Die in den Beispielen 43 bis 68 genannten Farbstoffe ergeben auf gebleichtem Zellstoff brillante Färbungen mit grünstichig gelben bis orangen Farbtönen.

Beispiel 69

Es wurden 6,4 g 1-Amino-4-benzoesäureanilid, wie in Beispiel 40 angegeben, diazotiert und mit 10,3 g 1,4-Bis[3-(4-methyl-6-hydroxypyrid-2-on-1-yl)imidazol-1-yliummethyl)]benzol-chlorid gekuppelt. Man erhielt 11 g eines gelben Farbstoffs der Formel

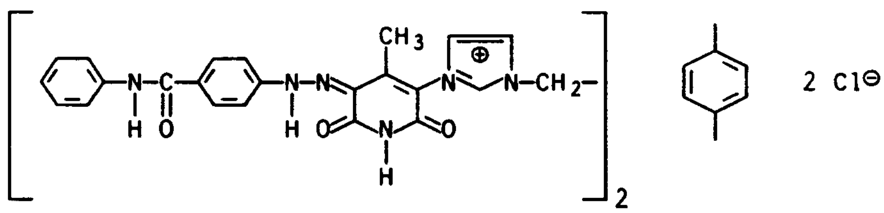

$\lambda_{max}$ (Essigsäure): 434 nm.

Beispiel 70

Anstelle des in Beispiel 69 angegebenen 1-Amino-4-benzosäureanilids wurden 6,4 g 4-Aminobenzoylaminobenzol verwendet. Man erhielt 8,6 g eines orangenen Farbstoffs der Formel

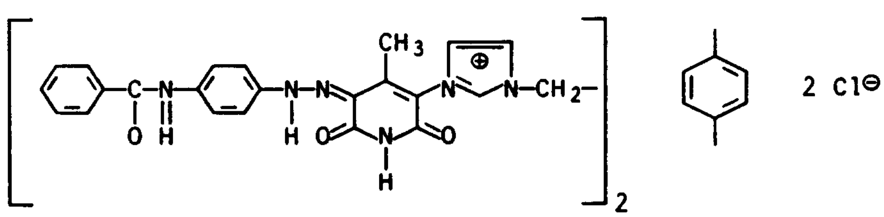

$\lambda_{max}$ (Essigsäure): 456 nm.

Beispiel 71 (Anwendung)

50 g Altpapier (holzhaltig) wurden in 1 l Wasser (10° dH) bei Raumtemperatur zu einer Fasersuspension aufgeschlagen. Anschließend wurde mit 1 l gleichem Wasser verdünnt. Der Suspension wurde eine Mischung aus 2 g einer 10 %igen essigsauren Lösung des Farbstoffs aus Beispiel 1 und 10 ml Wasser

zugesetzt. Das Gemisch wurde 15 Minuten leicht gerührt und dann mit Wasser auf 0,5 % Feststoffgehalt verdünnt. Mit dieser Suspension wurden auf einem Labor-Blattbildner der Firma Franck Papierblätter von 80 g/m$^2$ hergestellt und die feuchten Blätter 5 Minuten bei 100 °C getrocknet. Man erhielt ein gelbes, gefärbtes Schrenzpapier. Das Abwasser war farblos. Die Ausblutechtheiten des gefärbten Papieres (DIN 53 991) sind sehr gut, ebenso die Lichtechtheit.

Gleich gute Ergebnisse wurden erhalten, als das Altpapier durch a) eine Mischung auf 30 % Birkensulfatzellstoff und 70 % Kiefernsulfatzellstoff, durch b) Kiefernsulfitzellstoff und c) Kiefernsulfatzellstoff ersetzt wurde. Man erhielt in allen Fällen gelbe Papiere mit hervorragenden Echtheitseigenschaften.

Beispiel 72 (Anwendung)

15 kg Altpapier (holzhaltig), 25 kg gebleichter Holzschliff und 10 kg ungebleichter Sulfatzellstoff wurden im Pulper zu einer 3 %igen wäßrigen Stoffsuspension aufgeschlagen. Die Stoffsuspension wurde in einer Färbebütte auf 2 % verdünnt. Dieser Suspension wurden dann - gerechnet auf trockene Gesamtfaser - nacheinander unter Rühren 0,5 % lösliche, oxidativ abgebaute Maisstärke, 5 % Kaolin und 1,25 kg einer 5 %igen essigsauren Lösung des Farbstoffs aus Beispiel 13 zugegeben. Nach 20 Minuten wurde der Stoff in der Mischbütte mit 1 % (bezogen auf absolut trockene Faser) einer Harzleim-Dispersion versetzt. Die homogene Stoffsuspension wurde auf der Papiermaschine kurz vor dem Stoffauflauf mit Alaun auf einen pH-Wert von 5 eingestellt.

Auf der Papiermaschine wurde ein 80 g/m$^2$ schweres Tütenpapier maschinenglatt hergestellt, welches eine Orangenuance mit guten Ausblutechtheiten nach DIN 53 991 aufweist.

Beispiel 73 (Anwendung)

25 kg Katalogpapier (Ausschuß), 60 kg gebleichter Holzschliff (65° Schopper Riegler) und 15 kg ungebleichter Sulfitzellstoff wurden im Pulper in 2500 l Wasser aufgeschlagen. Der 4 %igen wäßrigen Stoffsuspension wurden 0,4 % lösliche Stärke, 16 % Kaolin und 2 % Talkum (berechnet auf absolut trockene Faser) zugegeben. Anschließend wurde die Stoffsuspension am Refiner auf 45° Schopper Riegler ausgemahlen. Der Stoffsuspension wurden 40 kg einer 3 %igen essigsauren Lösung des Farbstoffs aus Beispiel 40 zugesetzt (= 1 % trockener Farbstoff, bezogen auf absolut trockene Faser). Nach 15 Minuten Ziehzeit wurde dem Stoff Harzleim-Dispersion (Menge: 0,6 % trocken, bezogen auf absolut trockene Faser) zugesetzt. Nach 10 Minuten wurde der aus der Mischbütte abfließende Stoff kontinuierlich mit Wasser auf 0,8 % Stoffgehalt verdünnt und kontinuierlich mit Alaun ($Al_2(SO_4)_3 \cdot 18\ H_2O$) auf einen pH-Wert von 4,5 gestellt (gemessen im Siebwasser) und in den Stoffauflauf gepumpt.

Man erhielt goldgelbes Katalogpapier (60 g/m$^2$) mit guten Ausblutechtheiten.

**Patentansprüche**

**1.** Biskationische Azofarbstoffe der Formel I

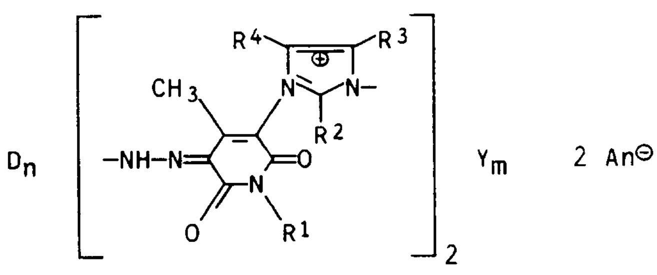

(I),

in der

| | |
|---|---|
| $R^1$ | Wasserstoff oder gegebenenfalls durch Phenyl substituiertes $C_1$-$C_5$-Alkyl, |
| $R^2$ | Wasserstoff oder $C_1$-$C_5$-Alkyl, |
| $R^3$ und $R^4$ | gleich oder verschieden sind und unabhängig voneinander jeweils Wasserstoff oder Methyl, |
| D | den Rest einer von Sulfonsäuregruppen freien aromatischen Diazokomponente, gegebenenfalls durch $C_1$-$C_4$-Alkyl oder $C_1$-$C_4$-Alkoxy substituiertes 1,4-Phenylen oder |

einen Rest der Formel

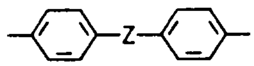

,

worin Z für ein Brückenglied steht,

Y Wasserstoff, $C_1$-$C_{18}$-Alkyl, $C_2$-$C_6$-Alkylen, Xyliden oder Phenylen,

$An^{\ominus}$ das Äquivalent eines Anions,

m 1 oder 2 und

n 1 oder 2 bedeuten,

mit der Maßgabe, daß

a) wenn Y Wasserstoff oder $C_1$-$C_{18}$-Alkyl bedeutet, m für 2, n für 1 und D für gegebenenfalls durch $C_1$-$C_4$-Alkyl oder $C_1$-$C_4$-Alkoxy substituiertes 1,4-Phenylen oder einen Rest der Formel

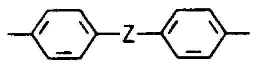

stehen, und

b) wenn Y $C_2$-$C_6$-Alkylen, Xyliden oder Phenylen bedeutet, m für 1, n für 2 und D für den Rest einer von Sulfonsäuregruppen freien aromatischen Diazokomponente stehen.

**2.** Biskationische Azofarbstoffe nach Anspruch 1, dadurch gekennzeichnet, daß

Y Wasserstoff oder $C_1$-$C_5$-Alkyl,

D Phenylen oder einen Rest der Formel

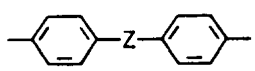

worin Z die in Anspruch 1 genannte Bedeutung besitzt,

m 2 und

n 1 bedeuten.

**3.** Biskationische Azofarbstoffe nach Anspruch 1, dadurch gekennzeichnet, daß

Y $C_2$-$C_6$-Alkylen, Xyliden oder Phenylen,

D den Rest einer von Sulfonsäuregruppen freien Diazokomponente aus der Anilinreihe,

m 1 und

n 2 bedeuten.

**4.** Verdoppelte Imidazolylpyridone der Formel II

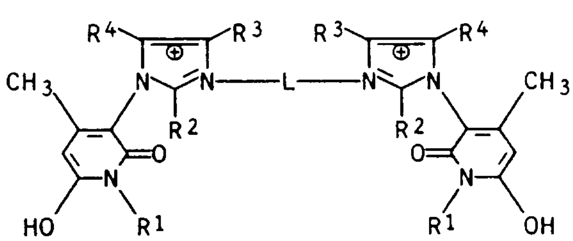

$2\ An^{\ominus}$ (II),

in der

L $C_2$-$C_6$-Alkylen, Xyliden oder Phenylen,

$R^1$ Wasserstoff oder gegebenenfalls durch Phenyl substituiertes $C_1$-$C_5$-Alkyl,

$R^2$ Wasserstoff oder $C_1$-$C_5$-Alkyl,

| | |
|---|---|
| $R^3$ und $R^4$ | gleich oder verschieden sind und unabhängig voneinander jeweils Wasserstoff oder Methyl und |
| $An^{\ominus}$ | das Äquivalent eines Anions bedeuten. |

**5.** Verwendung der biskationischen Azofarbstoffe gemäß Anspruch 1 zum Färben oder Bedrucken von polymerem Material.

## Claims

**1.** A biscationic azo dye of the formula I

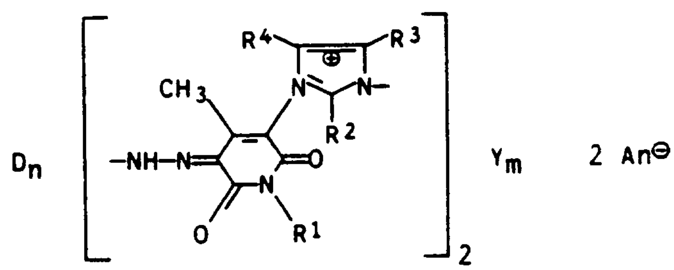

(I),

where

| | |
|---|---|
| $R^1$ | is hydrogen or unsubstituted or phenyl-substituted $C_1$-$C_5$-alkyl, |
| $R^2$ | is hydrogen or $C_1$-$C_5$-alkyl, |
| $R^3$ and $R^4$ | are identical or different and each is independently of the other hydrogen or methyl, |
| D | is the radical of a sulfo-devoid aromatic diazo component, unsubstituted or $C_1$-$C_4$-alkyl- or $C_1$-$C_4$-alkoxy-substituted 1,4-phenylene or a radical of the formula |

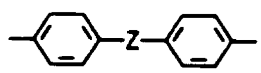

,

where Z is a bridge member,

| | |
|---|---|
| Y | is hydrogen, $C_1$-$C_{18}$-alkyl, $C_2$-$C_6$-alkylene, xylidene or phenylene, |
| $An^{\ominus}$ | is one equivalent of an anion, |
| m | is 1 or 2, and |
| n | is 1 or 2, |

with the proviso that

a) in the case of Y being hydrogen or $C_1$-$C_{18}$-alkyl, m is 2, n is 1 and D is unsubstituted or $C_1$-$C_4$-alkyl- or $C_1$-$C_4$-alkoxy-substituted 1,4-phenylene or a radical of the formula

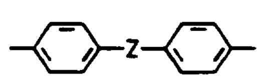

and

b) in the case of Y being $C_2$-$C_6$-alkylene, xylidene or phenylene, m is 1, n is 2 and D is the radical of a sulfo-devoid aromatic diazo component.

**2.** A biscationic azo dye as claimed in claim 1, wherein

| | |
|---|---|
| Y | is hydrogen or $C_1$-$C_5$-alkyl, |
| D | is phenylene or a radical of the formula |

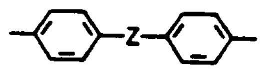

where Z is as defined in claim 1,
m is 2, and
n is 1.

**3.** A biscationic azo dye as claimed in claim 1, wherein
Y is $C_2$-$C_6$-alkylene, xylidene or phenylene,
D is the radical of a sulfo-devoid diazo component of the aniline series,
m is 1, and
n is 2.

**4.** A doubled imidazolylpyridone of the formula II

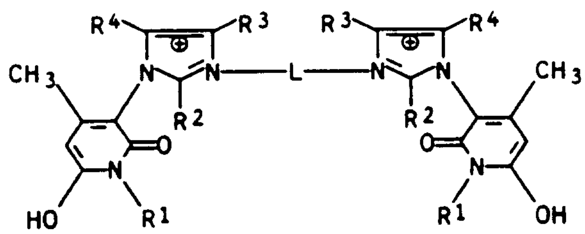

2 An$^{\ominus}$ (II),

where
L is $C_2$-$C_6$-alkylene, xylidene or phenylene,
$R^1$ is hydrogen or unsubstituted or phenyl-substituted $C_1$-$C_5$-alkyl,
$R^2$ is hydrogen or $C_1$-$C_5$-alkyl,
$R^3$ and $R^4$ are identical or different and each is independently of the other hydrogen or methyl, and
An$^{\ominus}$ is one equivalent of an anion.

**5.** Use of a biscationic azo dye as claimed in claim 1 for dyeing or printing polymeric material.

**Revendications**

**1.** Colorants azoïques biscationiques de formule I

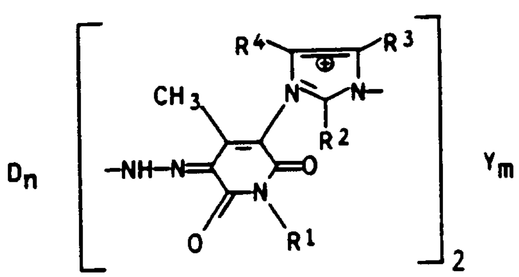

2 An$^{\ominus}$ (I),

dans laquelle
$R^1$ représente un atome d'hydrogène ou un reste alkyle en $C_1$-$C_5$ eventuellement substitué par un groupement phényle,
$R^2$ représente un atome d'hydrogène ou un reste alkyle en $C_1$-$C_5$,
$R^3$ et $R^4$ sont identiques ou différents et représentent chacun, indépendamment l'un de l'autre, un atome d'hydrogène ou un reste méthyle,

D est le reste d'un composant de diazoïque aromatique dépourvu de groupements acide sulfonique, un reste 1,4-phénylène éventuellement substitué par un groupement alkyle en $C_1$-$C_4$ ou alcoxy en $C_1$-$C_4$ ou un reste de formule

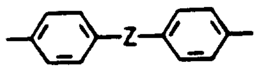 ,

où Z est mis pour un chaînon de pontage,
Y représente un atome d'hydrogène ou un reste alkyle en $C_1$-$C_{18}$, alkylène en $C_2$-$C_6$, xylidène ou phénylène,
$An^{\ominus}$ est l'équivalent d'un anion,
m est mis pour 1 ou 2 et
n est mis pour 1 ou 2,

étant spécifié que

a) quand Y représente un atome d'hydrogène ou un reste alkyle en $C_1$-$C_{18}$, m est mis pour 2, n pour 1 et D pour un reste 1,4-phénylène éventuellement substitué par un groupement alkyle en $C_1$-$C_4$ ou alcoxy en $C_1$-$C_4$ ou pour un reste de formule

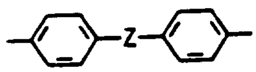

et

b) quand Y représente un reste alkylène en $C_2$-$C_6$, xylidène ou phénylène, m est mis pour 1, n pour 2 et D pour le reste d'un composant de diazoïque aromatique dépourvu de groupements acide sulfonique.

**2.** Colorants azoïques biscationiques selon la revendication 1, caractérises en ce que
Y représente un atome d'hydrogène ou un reste alkyle en $C_1$-$C_5$,
D représente un reste phénylène ou un reste de formule

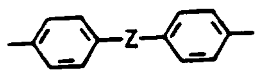

où Z a la signification donnée dans la revendication 1,
m est mis pour 2 et
n est mis pour 1.

**3.** Colorants azoïques biscationiques selon la revendication 1, caractérisés en ce que
Y représente un reste alkylène en $C_2$-$C_6$, xylidène ou phénylène,
D est le reste d'un composant de diazoïque de la série de l'aniline, dépourvu de groupements acide sulfonique,
m est mis pour 1 et
n est mis pour 2.

**4.** Imidazolylpyridones doublées de formule II

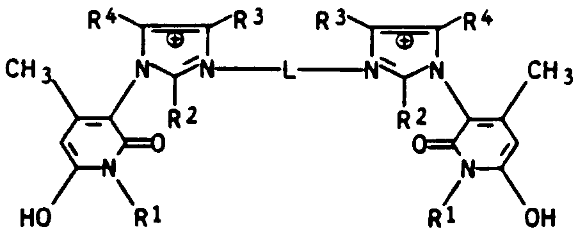

2 $An^{\ominus}$ (II),

dans laquelle

| | |
|---|---|
| L | représente un reste alkylène en $C_2$-$C_6$, xylidène ou phénylène, |
| $R^1$ | représente un atome d'hydrogène ou un reste alkyle en $C_1$-$C_5$ éventuellement substitué par un groupement phényle, |
| $R^2$ | représente un atome d'hydrogène ou un reste alkyle en $C_1$-$C_5$, |
| $R^3$ et $R^4$ | sont identiques ou différents et représentent chacun, indépendamment l'un de l'autre, un atome d'hydrogène ou un reste méthyle et |
| $An^{\ominus}$ | est l'équivalent d'un anion. |

**5.** Utilisation des colorants azoïques biscationiques selon la revendication 1 pour la teinture ou l'impression de matières polymères.